Europäisches Patentamt

European Patent Office

Office européen des brevets

(19)

(11) Publication number: **0 136 805 B1**

# (12) EUROPEAN PATENT SPECIFICATION

(45) Date of publication of patent specification: **09.10.91**   (51) Int. Cl.⁵: **C12P 7/18**

(21) Application number: **84305750.6**

(22) Date of filing: **22.08.84**

(54) **Industrial-scale process for the production of polyols by fermentation of sugars.**

(30) Priority: **24.08.83 GB 8322750**
**11.10.83 GB 8327191**

(43) Date of publication of application:
**10.04.85 Bulletin 85/15**

(45) Publication of the grant of the patent:
**09.10.91 Bulletin 91/41**

(84) Designated Contracting States:
**AT BE CH DE FR GB IT LI NL SE**

(56) References cited:

**ANTONIE VAN LEEUWENHOEK, vol. 37, 1971, pages 107-118; L. DOOMS et al.: "Polyol synthesis and taxonomic characters in the genus Moniliella"**

(73) Proprietor: **CPC INTERNATIONAL INC.**
**International Plaza P.O. Box 8000**
**Englewood Cliffs New Jersey 07632(US)**

(72) Inventor: **de Troostenbergh, Jean-Claude**
**Cleerbeekmolen 305**
**B-3215 Houwaart(BE)**
Inventor: **Avalosse, Bernard Léon Henri Marie**
**74, rue du Colmy**
**B-5544 Agimont(BE)**
Inventor: **Mignolet, René Louis**
**Martelarenlaan 183**
**B-3200 Kessel-Lo(BE)**

(74) Representative: **Wilkinson, Stephen John et al**
**Stevens, Hewlett & Perkins 1 St. Augustine's Place**
**Bristol BS1 4UD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The invention relates to an industrial-scale process for the production of glycerol and/or erythritol and/or ribitol, by aerobic fermentation of a sugar with a sugar-tolerant fungus such as Moniliella tomentosa var. pollinis.

It is known that aerobic fermentation of a suitable sugar by the yeast-like fungus Moniliella tomentosa var. pollinis will produce erythritol; this was first reported by G.J. Hajny, J.H. Smith and J.C. Garver in Applied Microbiology 12, pp 240-246 (May 1964), who designated the microbe as Torula I₂A. Later, in Antonie van Leeuwenhoek 37, pp 107-118 (1971), L. Dooms, G.L. Hennebert and H. Verachtert classified the fungus as Moniliella tomentosa var. pollinis, and confirmed its ability to produce erythritol; the authors also set forth a complete morphological description of the microbe.

In addition, they recognised that Moniliella can exist under two forms, yeast- and mould-like forms, according, essentially, to the applied culture conditions; on page 110 they state, "On agar slants both forms are produced. In stationary liquid media the mould-like form with abundant mycelium and more arthrospores than blastospores develops.

In shake-flask cultures, the yeast-like form predominates with rounded, oval and rectangular cells produced by budding and by fission while no mycelium is formed".

Moniliella tomentosa var. pollinis is available at the Centraal Bureau voor Schimmelcultures (CBS), Baarn, The Netherlands, as CBS 461.67, and was also deposited and is available to the public at the Commonwealth Mycological Institute Culture Collection (CMI cc), Ferry Lane, Kew, Richmond, Surrey TW9 3AF, United Kingdom, under the number (CMI cc) 271648.

In investigating the design of an industrial process for the production of polyols, especially erythritol and ribitol by the fermentation of sugars using Moniliella tomentosa var. pollinis, we have found that one form of the organism is particularly suitable for industrial use and we have devised a method whereby this form may be obtained.

Accordingly, therefore, the invention is an industrial process for the production of glycerol and/or erythritol and/or ribitol by the aerobic fermentation of a suitable sugar by Moniliella tomentosa var. pollinis in which the substrain of the Moniliella organism which is used has as high a spore-forming ability as possible.

We have found that when spores of Moniliella tomentosa var. pollinis are grown on a stationary medium e.g. agar, the colonies which are produced show different spore-forming abilities and that the different types of colony are detectable by their colour. Low spore-forming colonies are white in colour while high spore-forming colonies are black.

We have also found that when low spore-forming colonies (white colonies) of Moniliella tomentosa are used for the fermentation, a viscous polysaccharide is produced in the fermentation medium. Furthermore, as the viscosity of the medium increases, the oxygen transfer rate decreases. It has been shown by Hajny that in too low aeration conditions, Moniliella tomentosa converts part of the sugar to ethanol, thereby decreasing the polyol yield. Highly viscous culture conditions are, therefore, not favourable to polyol production. In addition, the Moniliella tomentosa cells are difficult to remove from the viscous medium and the polyol recovery is also very difficult. When the cultures are made from high spore-forming colonies (black colonies) very small amounts of polysaccharide are produced and higher polyol yields are obtained with consequentially lower ethanol production An additional advantage of the process of the invention is that black spores can easily be freeze-dried and stored for a long period of time.

In order to obtain the optimum spore-forming organism for use in the process according to the invention, spores of Moniliella tomentosa var. pollinis are grown on a suitable medium and selected spores from a "black colony" are used to inoculate a fresh medium and, after a suitable growing period, which may be up to 15 days, preferably at least seven days, at a temperature in the range 28°C to 32°C, preferably about 30°C, new black colonies are formed and a further selection is made to inoculate a second fresh growing medium. After preferably at least four such selections and growth stages (plating over), the spore-forming ability of the organism is stabilised.

The medium on which the organism is grown is suitably a mixture of malt extract 4%, yeast extract 2%, agar 2%, the balance being water.

Having selected a high spore-forming substrain of the Moniliella tomentosa var. pollinis, a pre-culture is made by cultivating the microorganism in a solution containing up to 20% (dry substance) of a suitable sugar such as dextrose, a nitrogen source (such as 0.5% yeast extract and 1% urea or 2% corn steep liquor) and 300 ppm Xanthan gum (see hereinafter) for 2 to 4 days. (Throughout the specification and claims, all percentages and parts are by weight to volume unless otherwise stated).

After this incubation and growth period, the culture is ready for use in the fermentation process where it is used to inoculate a medium containing sugar and a nitrogen source.

The desired products of the process according to the present invention are erythritol and/or ribitol. Prior art workers have reported production of glycerol and arabitol, in addition to erythritol, from the fermentation of sugars by Moniliella tomentosa var. pollinis. In accordance with our process, no arabitol is produced, but ribitol, in addition to erythritol and glycerol, is obtained, these three being the only polyols formed. Typically, the fermentation produces a solution product comprising ribitol, glycerol and erythritol in the following proportions, based on total polyols : ribitol 1%-20%; glycerol 5%-40%; the balance being erythritol.

Air is supplied to the fermentor at a rate which depends on the size of the fermentation vessel and the degree of agitation of the contents of the vessel but is generally in the range 0.1 to 1.5 litre air/litre fermentation medium/minute. For example, in a 2-litre vessel, the air flow rate was 1 litre/litre/minute at a stirrer speed of 400 to 800 rpm, while, in a 600-litre vessel, the rate was 0.5 to 1.0 litre/litre/minute without agitation other than that produced by the air flow. The fermentation is conducted at a temperature of between 27°C and 32°C at a starting pH of between 3 and 6, preferably 4 to 5. The fermentation is stopped when the sugar has been consumed. The amount of sugar contained in the medium can be between 20% and 45%, preferably 30% to 35%. Suitable sugars for use in both the culture and the fermentation are dextrose (glucose), sucrose, fructose or maltose.

In the prior art process, as described by Hajny, various sources of nitrogen were described for use in the fermentation, for example, yeast extract, urea, corn steep liquor, malt sprouts, black strap molasses, malt extract and distillers dry solubles. In the process of the present invention good results are achieved with 0.5% yeast extract plus 0.1% urea or with 2% corn steep liquor plus 0.02% urea.

The starting pH should be between about 3.0 and 6.0, which decreases to about 2.0 to 3.5 during the fermentation. According to L. Hanssens, A. Van Regenmortel and H. Verachtert, Applied Microbiology, Vol. 24, No. 5, pp 831-833 (November 1972) laboratory fermentations held at different constant pH's result in substantial differences in yields of total polyols and erythritol. We have found that, when working with larger scale fermentations (2-litre fermentors or larger) the total polyol and erythritol yields are less sensitive to starting pH within the 4.0 to 6.0 range. In order to avoid or minimise problems of contamination, a starting pH of 4 to 5 is preferred. The fermentation is conducted until all of the sugar has been consumed (fermentation times will normally be between 4 and 12 days, depending upon the amount of sugar used), after which the culture is stopped and the cells are removed from the culture broth, e.g. by centrifuging. The cell-free culture broth, which contains erythritol, ribitol and glycerol, can be used as such, with or without refining (e.g. by ultrafiltration and demineralisation), for certain applications, e.g. in the polymer industry. The refined culture broth can also be concentrated to 60% to 80% dissolved solids and the erythritol crystallised therefrom, e.g. by the technique described by J.M. Roxburg, J.F.T. Spencer and H.R. Sallens, Canadian Journal of Technology, Vol. 34 pp 248-253 (1956). The liquor remaining after recovery of the erythritol crystals, which is also a mixture of (non-crystallised) erythritol, ribitol and glycerol, can also be used after appropriate treatment.

The process according to the invention is advantageously carried out in the presence of the polysaccharide Xanthan gum which has the property of retaining the cells of the Moniliella tomentosa var. pollinis in the fermentation medium and reducing their loss via the foam which occurs during the fermentation. Preferably 100 to 500 ppm Xanthan gum may be present and excellent results are obtained with about 300 ppm. More gum may be used than is necessary for retaining the cells in the fermentation medium (i.e. more than about 500 ppm) but such excess is wasteful.

The overall process is also improved by including in the fermentation medium a conventional antifoam agent. Synthetic antifoam agents (e.g. silicone type or fatty alcohols) are preferred over the natural (e.g. lard oil) products because they can be used in smaller quantities and the final fermentation broth does not need extensive refining for their removal. With most synthetic commercial antifoam agents, 200-300 or 400 ppm is sufficient for optimum foam control.

The following examples are intended to illustrate the practice of the invention.

Examples

(a) Selection of Spores
Spores of Moniliella tomentosa var. pollinis were plated on Petri dishes containing the following medium :
Malt extract 4%
Yeast extract 2%
Agar 2%
The dishes were incubated at 30°C for 7 days. Colonies showing different spore formation abilities were detected from the colour of the colonies. Low spore forming colonies were white while high spore forming colonies were black. Spores from a black colony were used to inoculate new Petri dishes containing the same medium. After 7 days, new black colonies were selected and used for a new inoculation. After 4 successive "plating overs", the spore forming ability was stabilised.

(b) Preparation of the Seed Culture

500 ml erlenmeyers containing 50 ml culture medium comprising 20% dextrose, 0.5% yeast extract, 0.1% urea and 300 ppm Xanthan gum were inoculated with a colony from (a) above and cultivation was conducted for 3 to 4 days at a starting pH of 5, a temperature of 30°C and under reciprocating agitation of 100 oscillations/minute.

Example 1

Comparison of substrains

A black substrain exhibiting a high spore-forming ability was selected following (a) above. In the same manner, a white substrain exhibiting a low spore-forming ability was also selected and cultivated by a similar technique until a stable substrain was obtained. Both substrains were compared in shaken erlenmayer flasks in a medium comprising 32% dextrose, 0.5% yeast extract and 0.1% urea in two series of experiments (4 black and 4 white). The flasks were incubated for seven days and each member of both series was then analysed and was also used to inoculate a new fermentation medium. This procedure was repeated six times to give six successive sets of results. The four results in each set of the sequence were averaged and these results are given in the following Table :

| Set | High spore-forming substrain | | | Low spore-forming substrain | | |
| | Erythritol g/L | Ethanol g/L | Poly saccharide g/L | Erythritol g/L | Ethanol g/L | Poly saccharide g/L |
|---|---|---|---|---|---|---|
| 1 | 98 | 1.5 | 3.4 | 53 | 25 | 11 |
| 2 | 116 | 1.4 | 3.2 | 36 | 15 | 7.0 |
| 3 | 114 | 0.2 | 2.6 | 56 | 6.3 | 9.2 |
| 4 | 106 | 2.4 | 2.8 | 61 | 26 | 14 |
| 5 | 102 | 3.0 | 2.6 | 52 | 23 | 13 |
| 6 | 81 | 1.4 | 2.7 | 54 | 17 | 12 |

Fermentation Process

Example 2

A 2-litre fermentor containing 1.5 litre of a culture medium comprising 32% dextrose, 2% corn steep liquor (50% dissolved solids), 0.02% urea, 300 ppm SAG 471 antifoam (dimethylpolysiloxane from Union Carbide) and 300 ppm Xanthan gum was inoculated with the seed culture. The air flow rate was 1 litre air/litre fermentation medium/minute and the impeller speed 740 rpm. The fermentation was allowed to proceed for 11 days after which time the erythritol content of the fermentation medium was found to be 11% and the total polyol content 15%. The average daily dextrose consumption was 30g/litre/day.

Example 3

4

A 600-litre tower fermentation vessel containing 450 litres of a culture medium comprising 32% dextrose, 0.5% yeast extract, 0.1% urea, 300 ppm Xanthan gum and 300 ppm antifoam SAG 471 was inoculated with 5% of a seed culture. The starting pH was 5 and the air flow rate through the tower was 225 litres per minute. After 13 days, the final culture broth contained 10.8% erythritol, 5.6% glycerol, 1.7% ribitol and 0.8% dextrose. The erythritol yield was 34% and the total polyol yield was 56% of the consumed dextrose.

In a typical recovery operation cells were removed by centrifugation and the medium clarified by ultrafiltration and refined with ion exchangers. The colourless broth was concentrated to 70% dissolved solids and erythritol was crystallised from the liquor in the following manner. The temperature was brought to 65°C and then cooled, at a rate of 2°C per hour, to about room temperature. The crystals were recovered by filtration and washed once with ethanol. A typical crystallisation yield was 74% and the composition of the liquor remaining was erythritol, 132 g/l; glycerol, 152 g/l; ribitol, 79 g/l. In general, the composition of the liquor remaining after crystallisation of the erythritol may contain ribitol, glycerol and erythritol in the following proportions based on total polyols : ribitol, 5%-30%; glycerol, 30%-60%; the balance being erythritol. (These percentages being weight/weight).

## Claims

1. An industrial process for the production of glycerol and/or erythritol and/or ribitol, by the aerobic fermentation of a suitable sugar by Moniliella tomentosa var. pollinis characterised in that a substrain of the Moniliella organism is used which has as high a spore-forming ability as is possible.

2. A process according to Claim 1 characterised in that the substrain is chosen by cultivating spores of Moniliella tomentosa var. pollinis on a stationary medium and selecting from the colonies which develop those that are black as having the high spore-forming ability.

3. A process according to Claim 2 characterised in that the black colony selected is used to inoculate a new medium and from the colonies which grow, a further selection of a black colony is made.

4. A process according to Claim 3 in which the selection and growth process is repeated at least four times.

5. The process of any one of the preceding claims characterised in that air is fed to the fermentation at a flow rate between 0.5 and 1.0 litre/litre fermentation medium/minute.

6. The process of any one of the preceding claims characterised in that the sugar is dextrose.

7. The process of any one of the preceding claims characterised in that the sugar is present in the fermentation broth at the beginning of the fermentation in an amount of between 20% and 45%.

8. The process of any one of the preceding claims characterised in that the pH at the beginning of the fermentation is adjusted to between 3 and 6, preferably between 4 and 5.

9. The process of any one of the preceding claims characterised in that Xanthan gum is present in an amount between 100 ppm and 500 ppm of the fermentation medium.

10. The process of any one of the preceding claims characterised in that a conventional antifoam agent is also added to the fermentation.

## Revendications

1. Procédé industriel pour la production de glycérol et/ou d'érythritol et/ou de ribitol par fermentation aérobie d'un sucre convenable par Moniliella tomentosa var. pollinis, caractérisé en ce qu'on utilise une souche secondaire de l'organisme Moniliella ayant une capacité de sporulation aussi grande que possible.

2. Procédé suivant la revendication 1, caractérisé en ce qu'on choisit la souche secondaire par culture de spores de Moniliella tomentosa var. pollinis sur un milieu stationnaire, et on sélectionne parmi les

EP 0 136 805 B1

colonies qui se développent celles qui sont noires, comme colonies ayant une grande capacité de sporulation.

3. Procédé suivant la revendication 2, caractérisé en ce que la colonie noire sélectionnée est utilisée pour inoculer un nouveau milieu et parmi les colonies qui se développent, on procède à une nouvelle sélection d'une colonie noire.

4. Procédé suivant la revendication 3, dans lequel le processus de sélection et de croissance est répété au moins quatre fois.

5. Procédé suivant l'une quelconque des revendications précédentes, caractérisé en ce que de l'air est introduit dans le récipient de fermentation à une vitesse d'écoulement comprise entre 0,5 et 1,0 litre/litre de milieu de fermentation/minute.

6. Procédé suivant l'une quelconque des revendications précédentes, caractérisé en ce que le sucre est le dextrose.

7. Procédé suivant l'une quelconque des revendications précédentes, caractérisé en ce que le sucre est présent dans le bouillon de fermentation au début de la fermentation en une quantité comprise entre 20 et 45 %.

8. Procédé suivant l'une quelconque des revendications précédentes, caractérisé en ce que le pH au début de la fermentation est ajusté à une valeur comprise entre 3 et 6, de préférence entre 4 et 5.

9. Procédé suivant l'une quelconque des revendications précédentes, caractérisé en ce que de la gomme xanthane est présente en une quantité comprise entre 100 et 500 ppm par rapport au milieu de fermentation.

10. Procédé suivant l'une quelconque des revendications précédentes, caractérisé en ce qu'un agent anti-moussant classique est aussi ajouté au milieu de fermentation.

**Patentansprüche**

1. Verfahren zur Herstellung von Glycerin und/oder Erythrit und/oder Ribit in industriellem Umfang durch aerobe Gärung eines geeigneten Zuckers mit Moniliella tomentosa var. pollinis, dadurch gekennzeichnet, daß ein Unterstamm des Moniliella-Organismus verwendet wird, der eine möglichst große Fähigkeit zur Sporenbildung besitzt.

2. Verfahren nach Anspruch 1, dadurch gekennzeichnet, daß der Unterstamm ausgewählt wird, indem man Sporen von Moniliella tomentosa var. pollinis auf einem stationären Medium züchtet und aus den sich entwickelnden Kolonien solche auswählt, die schwarz sind, da sie eine viel Sporen bildende Fähigkeit besitzen.

3. Verfahren nach Anspruch 2, dadurch gekennzeichnet, daß die ausgewählte schwarze Kolonie zum Inokulieren eines neuen Mediums verwendet und von den wachsenden Kolonien eine weitere Selektion einer schwarzen Kolonie vorgenommen wird.

4. Verfahren nach Anspruch 3, dadurch gekennzeichnet, daß das Selektions- und Züchtungsverfahren mindestens vier Mal wiederholt wird.

5. Verfahren nach einem der vorhergehenden Ansprüche, dadurch gekennzeichnet, daß Luft mit einer Geschwindigkeit von 0.5 bis 1.0 Liter/Liter Gärmedium/Minute durch die Gärung geleitet wird.

6. Verfahren nach einem der vorhergehenden Ansprüche, dadurch gekennzeichnet, daß der Zucker Dextrose ist.

7. Verfahren nach einem der vorhergehenden Ansprüche, dadurch gekennzeichnet, daß der Zucker am Anfang der Gärung in der Gärbrühe in einer Menge zwischen 20% und 45% vorhanden ist.

6

8. Verfahren nach einem der vorhergehenden Ansprüche, dadurch gekennzeichnet, daß der pH am Anfang der Gärung auf 3 bis 6, vorzugsweise 4 bis 5, eingestellt wird.

9. Verfahren nach einem der vorhergehenden Ansprüche, dadurch gekennzeichnet, daß Xanthangummi in einer Menge von 100 ppm bis 500 ppm des Gärmediums vorhanden ist.

10. Verfahren nach einem der vorhergehenden Ansprüche, dadurch gekennzeichnet, daß der Gärung auch ein konventionelles Antischaummittel zugesetzt wird.